# EUROPEAN PATENT SPECIFICATION

(11) **EP 4 335 850 B1**
(45) Date of publication and mention of the grant of the patent: **09.07.2025**
(21) Application number: 22806648.6
(22) Date of filing: 07.05.2022
(51) Int. Cl.: C07D 519/00, C07C 309/30, A61P 35/00, A61K 31/53

(54) **SALT FORM OF PYRROLOTRIAZINE COMPOUND, CRYSTAL FORM THEREOF, AND PREPARATION METHOD THEREFOR**
SALZFORM EINER PYRROLOTRIAZINVERBINDUNG, KRISTALLFORM DAVON UND HERSTELLUNGSVERFAHREN DAFÜR
FORME SEL DE COMPOSÉ DE PYRROLOTRIAZINE, FORME CRISTALLINE DE CELUI-CI ET SON PROCÉDÉ DE PRÉPARATION

(30) Priority: 08.05.2021 CN 202110501179
(43) Date of publication of application: 13.03.2024
(73) Proprietor: Jumbo Drug Bank Co., Ltd., High-tech Zone Chengdu Sichuan 610000 (CN)
(72) Inventor: WU, Lingyun, Shanghai 200131 (CN); WEI, Xiawei, Chengdu, Sichuan 610000 (CN); YOU, Xu, Shanghai 200131 (CN); XU, Xiongbin, Shanghai 200131 (CN); JIANG, Ning, Chengdu, Sichuan 610000 (CN); CHEN, Shuhui, Shanghai 200131 (CN)
(74) Representative: Gille Hrabal Partnerschaftsgesellschaft mbB Patentanwälte
(86) International application number: PCT/CN2022/091452
(87) International publication number: WO 2022/237682

(56) References cited:
- EP-A1- 4 063 364
- WO-A1-2015/200481
- WO-A1-2017/087808
- WO-A1-2018/134148
- WO-A1-2018/134335
- WO-A1-2021/098691
- WO-A1-2021/098691
- CN-A- 106 795 162

## Description

### PRIORITY AND RELATED APPLICATIONS

The present disclosure claims priority to the prior application with the patent application No. 202110501179.9 and entitled "SALT FORM OF PYRROLOTRIAZINE COMPOUND, CRYSTAL FORM THEREOF, AND PREPARATION METHOD THEREFOR" filed to China National Intellectual Property Administration on May 8, 2021.

### TECHNICAL FIELD

The present disclosure relates to a salt form of a pyrrolotriazine compound, a crystal form thereof, a preparation method therefor, and use thereof, and in particular to a compound of formula (II) and a crystal form thereof.

### BACKGROUND

Mitogen-activated protein kinase interacting kinase (MAP kinase interacting kinase, or MNK for short) is a serine/threonine protein kinase. There are four subtypes for human MNK, namely MNK1a and MNK1b expressed by MNK1 gene, and MNK2a and MNK2b expressed by MNK2 gene, respectively. All the 4 subtypes contain a nuclear localization signal (NLS) sequence and a sequence for binding to eIF4G at the N-terminal, and are thus capable of entering into a cell nucleus to play a role, and recognizing and binding to downstream eIF4E. The subtypes MNK 1a and MNK2a have a binding site for MAPK at the C-terminal, and can be activated by upstream ERK and p38 phosphorylation. The nuclear export signal (NES) sequence at C-terminal of MNK1a makes it widely present in the cytoplasm, whereas the other 3 subtypes are mostly present in the nucleus.

Eukaryotic initiation factor 4E (eIF4E) is a cap-binding protein, and it can specifically recognize the cap structure at the 5' end of mRNA and is an important initiation factor in protein translation. S209-phosphorylated eIF4E can promote the translation of downstream proteins, mainly including c-MYC, cyclin D1, VEGF, FGF, and anti-apoptosis proteins such as mcl-1 and Bcl-2. The expression of eIF4E is up-regulated in lung cancer, colorectal cancer, gastric cancer, pancreatic duct carcinoma and other malignant tumors. MNK is the only kinase known to be able to phosphorylate eIF4E. In addition, MNK is located at an intersection of multiple signaling pathways involved in tumors and immune, such as RAS and T cell receptor (TCR), and can selectively control transcription of regulators of anti-tumor immune responses. MNK activity and activation of eIF4E are critical for tumor development and progression, but are not necessarily for normal cells. Therefore, a selective MNK inhibitor is expected to become an anti-tumor drug with low toxicity. EFT508 (WO2015/200481; WO2016/172010; WO2017/075394; WO2017/075412; WO2017/087808; WO2017/117052; WO2018/152117; WO2018/218038) is a selective oral MNK inhibitor being developed by EFFECTOR THERAPEUTICS INC. Research has shown that the eFT508 can selectively inhibit the expression of PD-1, LAG3 and IL-10, and improve the function of cytotoxic T cells without affecting the proliferation of normal T cells. Pre-clinical studies have found that the combination of eFT508 and PD-1 monoclonal antibody can enhance efficacy and increase response rate. The phase I clinical trial of the drug has been completed, and it showed good safety; currently, the monotherapy for hematological tumors and trending prostate cancer is in phase II clinical trial, the combined therapy with avelumab monoclonal antibody for microsatellite stable colorectal cancer (MSS CRC) is in phase II clinical trial, and the combined therapy with PD-1/PD-L1 therapy (for patients who are treated with PD-1/PD-L1 therapy alone before and have had disease progression or no complete or partial alleviation) for solid tumors is in phase II clinical trial. WO2018/134148 A1 and WO2018/134335A1 disclose compounds as MKNK1 and MKNK2 inhibitors.

### SUMMARY

The present disclosure provides a compound of formula (II),

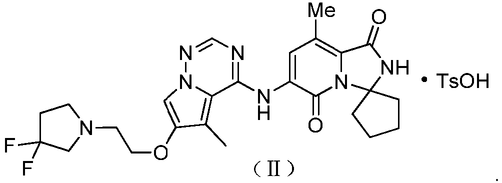

The present disclosure further provides crystal form A of the compound of formula (II), wherein an X-ray powder diffraction pattern of the crystal form A has characteristic diffraction peaks at the following 2θ angles: 7.78±0.20°, 11.38±0.20°, and 20.58±0.20°, when measured using a Cu Kα radiation;

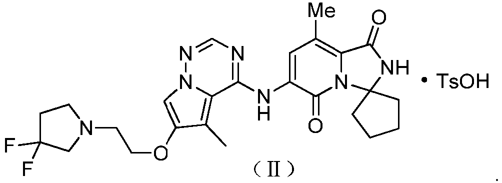

In some embodiments of the present disclosure, the X-ray powder diffraction pattern of the crystal form A described above has characteristic diffraction peaks at the following 2θ angles: 7.78±0.20°, 9.44±0.20°, 11.38±0.20°, 19.84±0.20°, 20.58±0.20°, 21.56±0.20°, 22.86±0.20°, and 24.82±0.20°.

**In** some embodiments of the present disclosure, the X-ray powder diffraction pattern of the crystal form A described above has characteristic diffraction peaks at the following 2θ angles: 6.58±0.20°, 7.78±0.20°, 9.44±0.20°, 11.38±0.20°, 14.38±0.20°, 18.66±0.20°, 19.84±0.20°, 20.58±0.20°, 21.56±0.20°, 22.86±0.20°, 23.54±0.20°, and 24.82±0.20°.

**In** some embodiments of the present disclosure, the X-ray powder diffraction pattern of the crystal form A described above has characteristic diffraction peaks at the following 20 angles: 4.78°±0.20°, 6.58°±0.20°, 7.78°±0.20°, 9.44°±0.20°, 11.38°±0.20°, 13.48°±0.20°, 14.38°±0.20°, 14.80°±0.20°, 16.42°±0.20°, 17.00°±0.20°, 17.32°±0.20°, 18.34°±0.20°, 18.66°±0.20°, 19.08°±0.20°, 19.60°±0.20°, 19.84°±0.20°, 20.28°±0.20°, 20.58°±0.20°, 21.56°±0.20°, 21.84°±0.20°, 22.52°±0.20°, 22.86°±0.20°, 23.26°±0.20°, 23.54°±0.20°, 24.46°±0.20°, 24.82°±0.20°, 25.50°±0.20°, 26.04°±0.20°, 26.58°±0.20°, 27.42°±0.20°, 27.82°±0.20°, 28.07°±0.20°, 28.42°±0.20°, 29.08°±0.20°, 29.66°±0.20°, 30.08°±0.20°, 31.20°±0.20°, 31.42°±0.20°, 38.22°±0.20°, and 39.04°±0.20°.

In some embodiments of the present disclosure, the X-ray powder diffraction pattern of the crystal form A described above has characteristic diffraction peaks at the following 20 angles: 4.78°±0.10°, 6.58°±0.10°, 7.78°±0.10°, 9.44°±0.10°, 11.38°±0.10°, 13.48°±0.10°, 14.38°±0.10°, 14.80°±0.10°, 16.42°±0.10°, 17.00°±0.10°, 17.32°±0.10°, 18.34°±0.10°, 18.66°±0.10°, 19.08°±0.10°, 19.60°±0.10°, 19.84°±0.10°, 20.28°±0.10°, 20.58°±0.10°, 21.56°±0.10°, 21.84°±0.10°, 22.52°±0.10°, 22.86°±0.10°, 23.26°±0.10°, 23.54°±0.10°, 24.46°±0.10°, 24.82°±0.10°, 25.50°±0.10°, 26.04°±0.10°, 26.58°±0.10°, 27.42°±0.10°, 27.82°±0.10°, 28.07°±0.10°, 28.42°±0.10°, 29.08°±0.10°, 29.66°±0.10°, 30.08°±0.10°, 31.20°±0.10°, 31.42°±0.10°, 38.22°±0.10°, and 39.04°±0.10°.

In some embodiments of the present disclosure, the X-ray powder diffraction pattern of the crystal form A described above has characteristic diffraction peaks at the following 2θ angles: 4.78°, 6.58°, 7.78°, 9.44°, 11.38°, 13.48°, 14.38°, 14.80°, 16.42°, 17.00°, 17.32°, 18.34°, 18.66°, 19.08°, 19.60°, 19.84°, 20.28°, 20.58°, 21.56°, 21.84°, 22.52°, 22.86°, 23.26°, 23.54°, 24.46°, 24.82°, 25.50°, 26.04°, 26.58°, 27.42°, 27.82°, 28.07°, 28.42°, 29.08°, 29.66°, 30.08°, 31.20°, 31.42°, 38.22°, and 39.04°.

In some embodiments of the present disclosure, the X-ray powder diffraction pattern of the crystal form A described above has characteristic diffraction peaks at the following 20 angles: 4.78°±0.20°, and/or 6.58°±0.20°, and/or 7.78°±0.20°, and/or 9.44°±0.20°, and/or 11.38°±0.20°, and/or 13.48°±0.20°, and/or 14.38°±0.20°, and/or 14.80°±0.20°, and/or 16.42°±0.20°, and/or 17.00°±0.20°, and/or 17.32°±0.20°, and/or 18.34°±0.20°, and/or 18.66°±0.20°, and/or 19.08°±0.20°, and/or 19.60°±0.20°, and/or 19.84°±0.20°, and/or 20.28°±0.20°, and/or 20.58°±0.20°, and/or 21.56°±0.20°, and/or 21.84°±0.20°, and/or 22.52°±0.20°, and/or 22.86°±0.20°, and/or 23.26°±0.20°, and/or 23.54°±0.20°, and/or 24.46°±0.20°, and/or 24.82°±0.20°, and/or 25.50°±0.20°, and/or 26.04°±0.20°, and/or 26.58°±0.20°, and/or 27.42°±0.20°, and/or 27.82°±0.20°, and/or 28.07°±0.20°, and/or 28.42°±0.20°, and/or 29.08°±0.20°, and/or 29.66°±0.20°, and/or 30.08°±0.20°, and/or 31.20°±0.20°, and/or 31.42°±0.20°, and/or 38.22°±0.20°, and/or 39.04°±0.20°.

In some embodiments of the present disclosure, the X-ray powder diffraction pattern of the crystal form A described above has characteristic diffraction peaks at the following 2θ angles: 4.78°±0.10°, and/or 6.58°±0.10°, and/or 7.78°±0.10°, and/or 9.44°±0.10°, and/or 11.38°±0.10°, and/or 13.48°±0.10°, and/or 14.38°±0.10°, and/or 14.80°±0.10°, and/or 16.42°±0.10°, and/or 17.00°±0.10°, and/or 17.32°±0.10°, and/or 18.34°±0.10°, and/or 18.66°±0.10°, and/or 19.08°±0.10°, and/or 19.60°±0.10°, and/or 19.84°±0.10°, and/or 20.28°±0.10°, and/or 20.58°±0.10°, and/or 21.56°±0.10°, and/or 21.84°±0.10°, and/or 22.52°±0.10°, and/or 22.86°±0.10°, and/or 23.26°±0.10°, and/or 23.54°±0.10°, and/or 24.46°±0.10°, and/or 24.82°±0.10°, and/or 25.50°±0.10°, and/or 26.04°±0.10°, and/or 26.58°±0.10°, and/or 27.42°±0.10°, and/or 27.82°±0.10°, and/or 28.07°±0.10°, and/or 28.42°±0.10°, and/or 29.08°±0.10°, and/or 29.66°±0.10°, and/or 30.08°±0.10°, and/or 31.20°±0.10°, and/or 31.42°±0.10°, and/or 38.22°±0.10°, and/or 39.04°±0.10°.

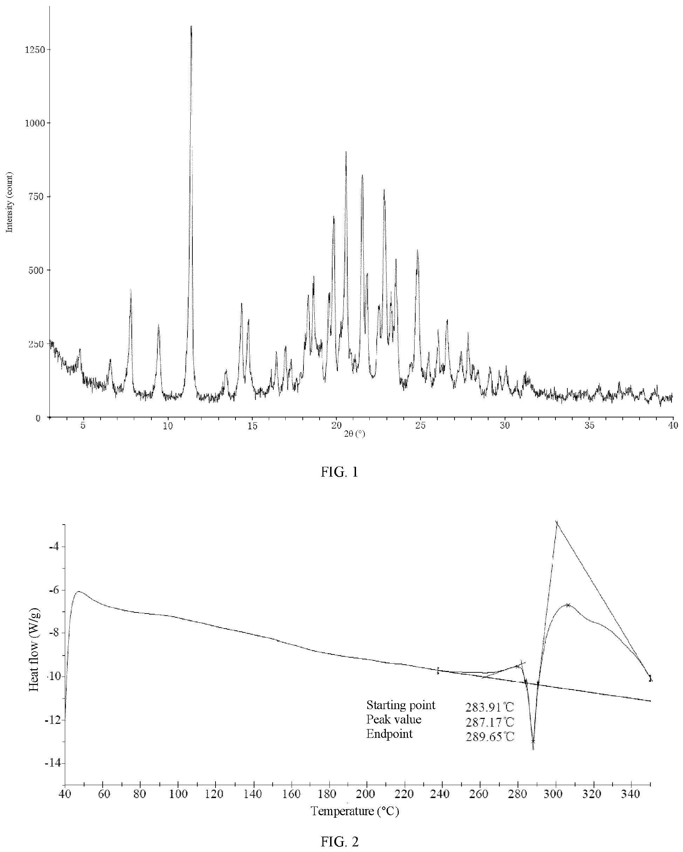

In some embodiments of the present disclosure, the XRPD pattern of the crystal form A described above is as shown in FIG. 1.

In some embodiments of the present disclosure, XRPD pattern analysis data for the crystal form A described above are shown in Table 1.

**Table 1. XRPD analysis data for crystal form A of compound of formula (II)**

| No. | 2θ angle (°) | Peak intensity (cts) | Interplanar spacing (Å) | Relative intensity (%) | No. | 2θ angle (°) | Peak intensity (cts) | Interplanar spacing (Å) | Relative intensity (%) |
|---|---|---|---|---|---|---|---|---|---|
| 1 | 4.78 | 124 | 18.46 | 9.8 | 21 | 22.52 | 240 | 3.94 | 19.0 |
| 2 | 6.58 | 104 | 13.42 | 8.2 | 22 | 22.86 | 640 | 3.89 | 50.6 |
| 3 | 7.78 | 357 | 11.35 | 28.2 | 23 | 23.26 | 301 | 3.82 | 23.8 |
| 4 | 9.44 | 245 | 9.36 | 19.3 | 24 | 23.54 | 426 | 3.78 | 33.6 |
| 5 | 11.38 | 1265 | 7.77 | 100.0 | 25 | 24.46 | 68 | 3.64 | 5.4 |
| 6 | 13.48 | 93 | 6.57 | 7.3 | 26 | 24.82 | 453 | 3.58 | 35.8 |
| 7 | 14.38 | 310 | 6.15 | 24.5 | 27 | 25.50 | 93 | 3.49 | 7.4 |
| 8 | 14.80 | 251 | 5.98 | 19.8 | 28 | 26.04 | 179 | 3.42 | 14.1 |
| 9 | 16.42 | 137 | 5.39 | 10.9 | 29 | 26.58 | 214 | 3.35 | 16.9 |
| 10 | 17.00 | 144 | 5.21 | 11.4 | 30 | 27.42 | 97 | 3.25 | 7.7 |
| 11 | 17.32 | 91 | 5.12 | 7.2 | 31 | 27.82 | 190 | 3.20 | 15.0 |
| 12 | 18.34 | 291 | 4.83 | 23.0 | 32 | 28.07 | 85 | 3.18 | 6.7 |
| 13 | 18.66 | 349 | 4.75 | 27.6 | 33 | 28.42 | 81 | 3.14 | 6.4 |
| 14 | 19.08 | 136 | 4.65 | 10.8 | 34 | 29.08 | 91 | 3.07 | 7.2 |
| 15 | 19.60 | 269 | 4.53 | 21.2 | 35 | 29.66 | 81 | 3.01 | 6.4 |
| 16 | 19.84 | 514 | 4.47 | 40.6 | 36 | 30.08 | 90 | 2.97 | 7.1 |
| 17 | 20.28 | 167 | 4.38 | 13.2 | 37 | 31.20 | 71 | 2.86 | 5.6 |
| 18 | 20.58 | 726 | 4.31 | 57.4 | 38 | 31.42 | 63 | 2.84 | 5.0 |
| 19 | 21.56 | 667 | 4.12 | 52.7 | 39 | 38.22 | 36 | 2.35 | 2.8 |
| 20 | 21.84 | 341 | 4.07 | 26.9 | 40 | 39.04 | 57 | 2.31 | 4.5 |

In some embodiments of the present disclosure, a differential scanning calorimetry curve of the crystal form A described above has an endothermic peak at 287.17±3 °C, when measured using scanning speed of 10°C per minute.

In some embodiments of the present disclosure, a DSC profile of the crystal form A described above is shown in FIG. 2, when measured using scanning speed of 10°C per minute.

In some embodiments of the present disclosure, a thermogravimetric analysis curve (TGA) of the crystal form A described above shows a weight loss of 0.075% at 200.0±3 °C, when measured using scanning speed of 10°C per minute.

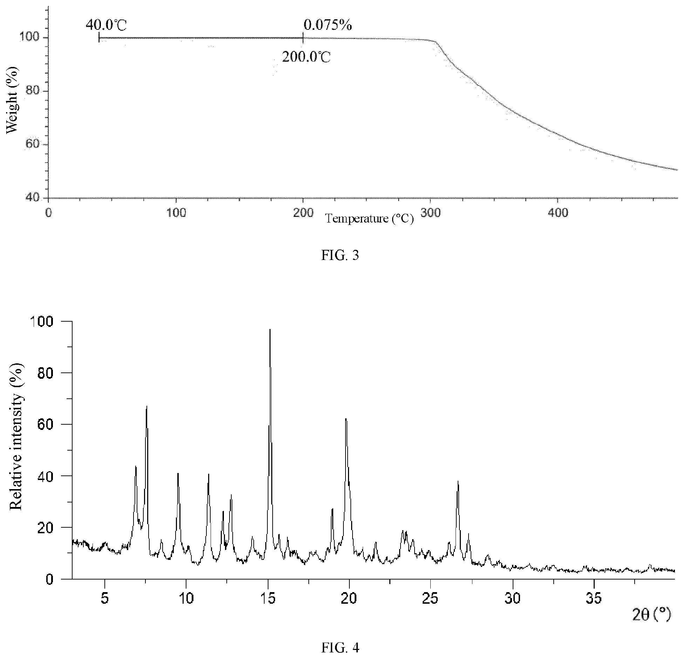

In some embodiments of the present disclosure, a TGA profile of the crystal form A described above is shown in FIG. 3, when measured using scanning speed of 10°C per minute.

The present disclosure further provides a preparation method for the crystal form A of the compound of formula (II), comprising the following steps:
(a) adding the compound of formula (II) to a solvent to form a suspension;
(b) stirring the suspension at 40-55 °C for 2-25 h; and
(c) filtering the suspension and then drying the filter cake in vacuum at 30-45 °C for 10-24 h;
wherein the solvent is selected from methanol, acetonitrile, and *tert-butyl* methyl ether.

The present disclosure provides crystal form B of the compound of formula (II), wherein an X-ray powder diffraction (XRPD) pattern of the crystal form B has characteristic diffraction peaks at the following 2θ angles: 7.55±0.20°, 15.13±0.20°, and 19.82±0.20°, when measured using a Cu Kα radiation;

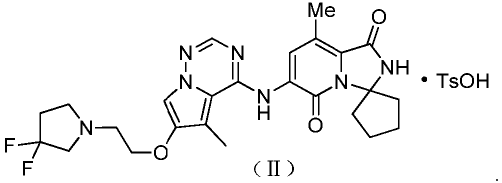

In some embodiments of the present disclosure, the X-ray powder diffraction pattern of the crystal form B described above has characteristic diffraction peaks at the following 2θ angles: 6.89±0.20°, 7.55±0.20°, 9.50±0.20°, 11.35±0.20°, 12.72±0.20°, 15.13±0.20°, 19.82±0.20°, and 26.63±0.20°.

In some embodiments of the present disclosure, the X-ray powder diffraction pattern of the crystal form B described above has characteristic diffraction peaks at the following 2θ angles: 6.89±0.20°, 7.55±0.20°, 9.50±0.20°, 11.35±0.20°, 12.24±0.20°, 12.72±0.20°, 15.13±0.20°, 18.94±0.20°, 19.82±0.20°, 23.25±0.20°, 26.63±0.20°, and 27.27±0.20°.

In some embodiments of the present disclosure, the X-ray powder diffraction pattern of the crystal form B described above has characteristic diffraction peaks at the following 20 angles: 4.98°±0.20°, 6.89°±0.20°, 7.55°±0.20°, 8.46°±0.20°, 9.50°±0.20°, 10.12°±0.20°, 11.35°±0.20°, 12.24°±0.20°, 12.72°±0.20°, 14.05°±0.20°, 15.13°±0.20°, 15.65°±0.20°, 16.20°±0.20°, 17.79°±0.20°, 18.94°±0.20°, 19.82°±0.20°, 20.76°±0.20°, 21.61°±0.20°, 23.25°±0.20°, 23.87°±0.20°, 26.09°±0.20°, 26.63°±0.20°, 27.27°±0.20°, 28.45°±0.20°, 29.12°±0.20°, 30.95°±0.20°, 32.32°±0.20°, 34.62°±0.20°, and 38.41°±0.20°.

**In** some embodiments of the present disclosure, the X-ray powder diffraction pattern of the crystal form B described above has characteristic diffraction peaks at the following 20 angles: 4.98°±0.10°, 6.89°±0.10°, 7.55°±0.10°, 8.46°±0.10°, 9.50°±0.10°, 10.12°±0.10°, 11.35°±0.10°, 12.24°±0.10°, 12.72°±0.10°, 14.05°±0.10°, 15.13°±0.10°, 15.65°±0.10°, 16.20°±0.10°, 17.79°±0.10°, 18.94°±0.10°, 19.82°±0.10°, 20.76°±0.10°, 21.61°±0.10°, 23.25°±0.10°, 23.87°±0.10°, 26.09°±0.10°, 26.63°±0.10°, 27.27°±0.10°, 28.45°±0.10°, 29.12°±0.10°, 30.95°±0.10°, 32.32°±0.10°, 34.62°±0.10°, and 38.41°±0.10°.

**In** some embodiments of the present disclosure, the X-ray powder diffraction pattern of the crystal form B described above has characteristic diffraction peaks at the following 2θ angles: 4.98°, 6.89°, 7.55°, 8.46°, 9.50°, 10.12°, 11.35°, 12.24°, 12.72°, 14.05°, 15.13°, 15.65°, 16.20°, 17.79°, 18.94°, 19.82°, 20.76°, 21.61°, 23.25°, 23.87°, 26.09°, 26.63°, 27.27°, 28.45°, 29.12°, 30.95°, 32.32°, 34.62°, and 38.41°.

**In** some embodiments of the present disclosure, the X-ray powder diffraction pattern of the crystal form B described above has characteristic diffraction peaks at the following 20 angles: 4.98°±0.20°, and/or 6.89°±0.20°, and/or 7.55°±0.20°, and/or 8.46°±0.20°, and/or 9.50°±0.20°, and/or 10.12°±0.20°, and/or 11.35°±0.20°, and/or 12.24°±0.20°, and/or 12.72°±0.20°, and/or 14.05°±0.20°, and/or 15.13°±0.20°, and/or 15.65°±0.20°, and/or 16.20°±0.20°, and/or 17.79°±0.20°, and/or 18.94°±0.20°, and/or 19.82°±0.20°, and/or 20.76°±0.20°, and/or 21.61°±0.20°, and/or 23.25°±0.20°, and/or 23.87°±0.20°, and/or 26.09°±0.20°, and/or 26.63°±0.20°, and/or 27.27°±0.20°, and/or 28.45°±0.20°, and/or 29.12°±0.20°, and/or 30.95°±0.20°, and/or 32.32°±0.20°, and/or 34.62°±0.20°, and/or 38.41°±0.20°.

**In** some embodiments of the present disclosure, the X-ray powder diffraction pattern of the crystal form B described above has characteristic diffraction peaks at the following 20 angles: 4.98°±0.10°, and/or 6.89°±0.10°, and/or 7.55°±0.10°, and/or 8.46°±0.10°, and/or 9.50°±0.10°, and/or 10.12°±0.10°, and/or 11.35°±0.10°, and/or 12.24°±0.10°, and/or 12.72°±0.10°, and/or 14.05°±0.10°, and/or 15.13°±0.10°, and/or 15.65°±0.10°, and/or 16.20°±0.10°, and/or 17.79°±0.10°, and/or 18.94°±0.10°, and/or 19.82°±0.10°, and/or 20.76°±0.10°, and/or 21.61°±0.10°, and/or 23.25°±0.10°, and/or 23.87°±0.10°, and/or 26.09°±0.10°, and/or 26.63°±0.10°, and/or 27.27°±0.10°, and/or 28.45°±0.10°, and/or 29.12°±0.10°, and/or 30.95°±0.10°, and/or 32.32°±0.10°, and/or 34.62°±0.10°, and/or 38.41°±0.10°.

In some embodiments of the present disclosure, the XRPD pattern of the crystal form B described above is as shown in FIG. 4.

In some embodiments of the present disclosure, XRPD pattern analysis data for the crystal form B described above are shown in Table 2.

**Table 2. XRPD analysis data for crystal form B of compound of formula (II)**

| No. | 2θ angle (°) | Peak intensity (cts) | Interplanar spacing (Å) | Relative intensity (%) | No. | 2θ angle (°) | Peak intensity (cts) | Interplanar spacing (Å) | Relative intensity (%) |
|---|---|---|---|---|---|---|---|---|---|
| 1 | 4.98 | 72.32 | 17.74 | 2.97 | 16 | 19.82 | 1532.59 | 4.48 | 62.93 |
| 2 | 6.89 | 896.22 | 12.82 | 36.80 | 17 | 20.76 | 150.45 | 4.28 | 6.18 |
| 3 | 7.55 | 1530.49 | 11.71 | 62.85 | 18 | 21.61 | 244.84 | 4.11 | 10.05 |
| 4 | 8.46 | 157.42 | 10.46 | 6.46 | 19 | 23.25 | 347.03 | 3.83 | 14.25 |
| 5 | 9.50 | 859.72 | 9.31 | 35.30 | 20 | 23.87 | 274.20 | 3.73 | 11.26 |
| 6 | 10.12 | 135.06 | 8.74 | 5.55 | 21 | 26.09 | 263.02 | 3.42 | 10.80 |
| 7 | 11.35 | 889.45 | 7.80 | 36.52 | 22 | 26.63 | 913.50 | 3.35 | 37.51 |
| 8 | 12.24 | 510.42 | 7.23 | 20.96 | 23 | 27.27 | 351.00 | 3.27 | 14.41 |
| 9 | 12.72 | 666.73 | 6.96 | 27.38 | 24 | 28.45 | 138.33 | 3.14 | 5.68 |
| 10 | 14.05 | 259.16 | 6.31 | 10.64 | 25 | 29.12 | 70.84 | 3.07 | 2.91 |
| 11 | 15.13 | 2435.25 | 5.86 | 100.00 | 26 | 30.95 | 54.09 | 2.89 | 2.22 |
| 12 | 15.65 | 242.34 | 5.66 | 9.95 | 27 | 32.32 | 32.02 | 2.77 | 1.31 |
| 13 | 16.20 | 272.35 | 5.47 | 11.18 | 28 | 34.62 | 16.98 | 2.59 | 0.70 |
| 14 | 17.79 | 95.51 | 4.99 | 3.92 | 29 | 38.41 | 68.55 | 2.34 | 2.81 |
| 15 | 18.94 | 579.19 | 4.69 | 23.78 | | | | | |

In some embodiments of the present disclosure, a differential scanning calorimetry curve of the crystal form B described above has an endothermic peak at 300.0±3 °C, when measured using scanning speed of 10°C per minute.

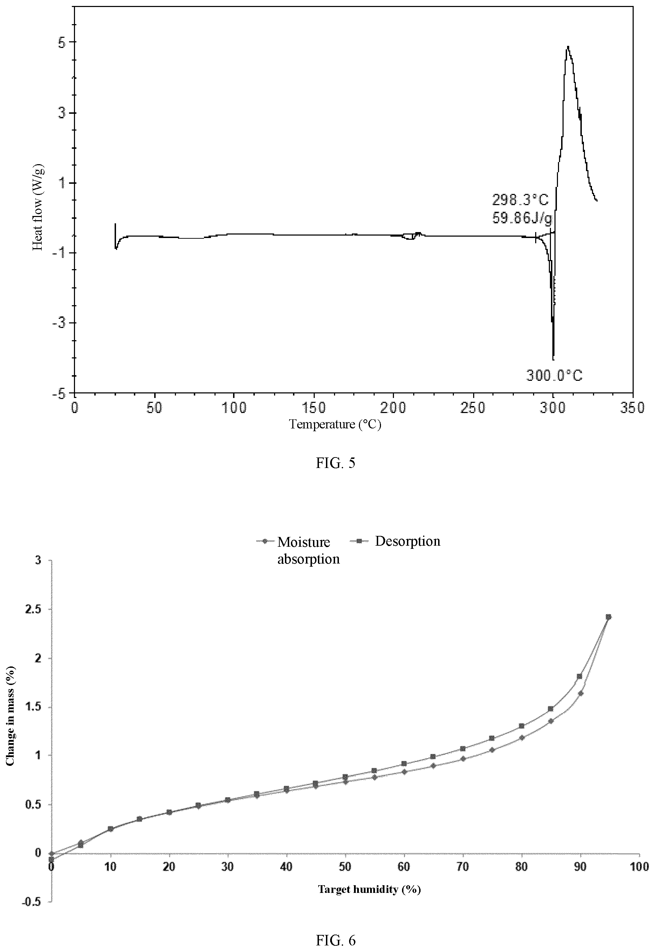

In some embodiments of the present disclosure, a DSC profile of the crystal form B described above is shown in FIG. 5, when measured using scanning speed of 10°C per minute.

The present disclosure further provides a preparation method for the crystal form B of the compound of formula (II), comprising the following steps:
(a) adding the compound of formula (II) to a solvent to form a suspension;
(b) stirring the suspension at 40-55 °C for 2-25 h; and
(c) filtering the suspension and then drying the filter cake in vacuum at 30-45 °C for 10-24 h;
wherein the solvent is selected from ethanol and n-heptane.

The present disclosure further provides the compound of formula (II) described above, the crystal form A described above or the crystal form B described above for use as a medicament.

The present disclosure further provides the compound of formula (II) described above, the crystal form A described above or the crystal form B described above for use as an MNK1/2 inhibitor medicament.

The present disclosure further provides the compound of formula (II) described above, the crystal form A described above and the crystal form B described above for use in treatmentment of colorectal cancer.

### Technical Effects

The compound of formula (I) of the present disclosure has high selectivity for MNK1/2 and also has significant inhibitory activity against the kinases; in addition, the compound has good membrane permeability and has excellent pharmacokinetic and pharmacodynamic properties. Each of crystal forms of the compound of formula (II) is stable, has little influence by light, heat and humidity, and has good *in-vivo* efficacy and a wide pharmaceutical prospect.

### Definitions and Description

Unless otherwise stated, the following terms and phrases used herein are intended to have the following meanings. A particular phrase or term, unless otherwise specifically defined, should not be considered as uncertain or unclear, but construed according to its common meaning. When referring to a trade name, it is intended to refer to its corresponding commercial product or its active ingredient.

The intermediate compounds of the present disclosure can be prepared by a variety of synthetic methods well known to those skilled in the art, including the specific embodiments listed below, embodiments formed by combinations thereof with other chemical synthetic methods, and equivalent substitutions thereof known to those skilled in the art.

The chemical reactions of the specific embodiments of the present disclosure are carried out in a suitable solvent that must be suitable for the chemical changes in the present disclosure and the reagents and materials required. In order to acquire the compounds of the present disclosure, it is sometimes necessary for those skilled in the art to modify or select a synthesis procedure or a reaction scheme based on the existing embodiments.

The structures of the compounds of the present disclosure can be confirmed by conventional methods well known to those skilled in the art, and if the present disclosure relates to an absolute configuration of the compound, the absolute configuration can be confirmed by means of conventional techniques in the art. For example, in the single crystal X-ray diffraction (SXRD) method, intensity data of diffraction of the single crystal grown are collected with a Bruker D8 venture diffractometer, the light source is CuKα radiation, and the scanning mode is φ/ω scanning; after related data are collected, the direct method (Shelxs97) is further employed to analyze the crystal structure, and thus the absolute configuration can be confirmed.

The present disclosure is described in detail below by way of examples, which are not intended to limit the present disclosure in any way.

All solvents used in the present disclosure are commercially available and can be used without further purification. The following abbreviations are used in the present disclosure: DCM represents dichloromethane; DMF represents *N,N*-dimethylformamide; DMSO represents dimethyl sulfoxide; EtOH represents ethanol; MeOH represents methanol; 2-MeTHF represents 2-methyltetrahydrofuran; ACN represents acetonitrile; toluene represents methylbenzene; EtOAc represents ethyl acetate; THF represents tetrahydrofuran; H₂O represents water; TosOH represents p-toluenesulfonic acid.

Compounds are named according to conventional nomenclature rules in the art or using ChemDraw^{®} software, and supplier's catalog names are given for commercially available compounds.

### BRIEF DESCRIPTION OF THE DRAWINGS

FIG. 1 shows an XRPD pattern of crystal form A of a compound of formula (II);
FIG. 2 shows a DSC profile of the crystal form A of the compound of formula (II);
FIG. 3 shows a TGA profile of the crystal form A of the compound of formula (II);
FIG. 4 shows an XRPD pattern of crystal form B of the compound of formula (II);
FIG. 5 shows a DSC profile of the crystal form B of the compound of formula (II);
FIG. 6 shows a DVS profile of the crystal form A of the compound of formula (II); and
FIG. 7 shows an ellipsoid plot of a single crystal of the compound of formula (II).

### Instrument parameters

**The X-ray powder diffractometer (XRPD) method for the crystal form A of the present disclosure has test parameters shown in Table 3.**

**Table 3. XRPD test parameters for crystal form A**

| XRPD test parameters | |
|---|---|
| Parameter | Set value |
| Model | DX-2700BH |
| X-ray | Cu, k-α: 1.54184Å; |
| X-ray light tube settings | 40 kV, 30 mA |
| Divergence slit | 1 mm |
| Primary scattering slit | 28 mm |
| Secondary slit | 28 mm |
| Detector slit | 0.3 mm |
| Anti-scatter slit | 1 mm |
| Scanning axis | θs-θd |
| Scanning range 2θ (°) | 3~40 |
| Scanning time (s) | 0.5 |
| Scanning step size 2θ (°) | 0.02 |

**The differential scanning calorimeter (DSC) method for the crystal form A of the present disclosure has test parameters shown in Table 4.**

**Table 4. DSC test parameters for crystal form A**

| DSC test parameters | |
|---|---|
| Model of instrument | Mettler DSC1 |
| Method | Linear heating |
| Crucible type | High-pressure gold-plated crucible, closed |
| Temperature range | 40-350°C |
| Scanning speed (°C/min) | 10 |
| Protective gas | Nitrogen |

**The thermal gravimetric analyzer (TGA) method for the crystal form A of the present disclosure has test parameters shown in Table 5.**

**Table 5. TGA test parameters for crystal form A**

| TGA test parameters | |
|---|---|
| Model of instrument | TGA550 |
| Method | Linear heating |
| Sample tray | Aluminum tray, open |
| Temperature range | 40~500°C |
| Scanning speed (°C/min) | 10 |
| Protective gas | Nitrogen |

**The dynamic vapor sorption (DVS) method for the crystal form A of the present disclosure has test parameters shown in Table 6.**

**Table 6. DVS test parameters for crystal form A**

| | |
|---|---|
| Manufacturer and model of instrument | SMS/ DVS intrinsic |
| Test condition | Weighing about 10 mg of sample for testing |
| Temperature | 25°C |
| Equilibration | dm/dt: 0.01%/min |
| Drying | Drying at 25 °C and 0% RH for 2 h |
| RH (%) test gradient | 5% RH |
| Range of RH (%) test gradient | 0%~95%~0% RH |

**The evaluation categories of hygroscopicity are shown in Table 7 below:**

**Table 7. Evaluation category table of hygroscopicity**

| **Categories of hygroscopicity** | **ΔW%** |
|---|---|
| Deliquescence | Absorbing sufficient water to form a solution |
| Very hygroscopic | ΔW% ≥ 15% |
| Hygroscopic | 15% > ΔW% ≥ 2% |
| Slightly hygroscopic | 2% > ΔW% ≥ 0.2% |
| Non-hygroscopic or hardly hygroscopic | ΔW% < 0.2% |

| | |
|---|---|
| Note: ΔW% represents the hygroscopic weight gain of the test compound at 25±1 °C and 80±2% RH. | |

**The X-ray powder diffractometer (XRPD) method for the crystal form B of the present disclosure has test parameters shown in Table 8.**

**Table 8. XRPD test parameters for crystal form B**

| XRPD test parameters | |
|---|---|
| Parameter | Set value |
| Model | X'Pert³ |
| X-ray | Cu, kα, Kα1 (Å): 1.54060; Kα2 (Å): 1.54443; |
| X-ray light tube settings | 45 kV, 40 mA |
| Divergence slit | Fixed 1/8° |
| Anti-scatter slit | P7.5 |
| Scanning mode | Continuous |
| Scanning range 20 (°) | 3~40 |
| Scanning time of each step (s) | 46.665 |
| Scanning step size 20 (°) | 0.0263 |

**The differential scanning calorimeter (DSC) method for the crystal form B of the present disclosure has test parameters shown in Table 9.**

**Table 9. DSC test parameters for crystal form B**

| DSC test parameters | |
|---|---|
| Model of instrument | TA Discovery DSC 2500 |
| Method | Linear heating |
| Sample tray | Aluminum tray, closed |
| Temperature range | 25-330°C |
| Scanning speed (°C/min) | 10 |
| Protective gas | Nitrogen |

### Example 1: Preparation of Compound of Formula (I)

### Synthetic route:

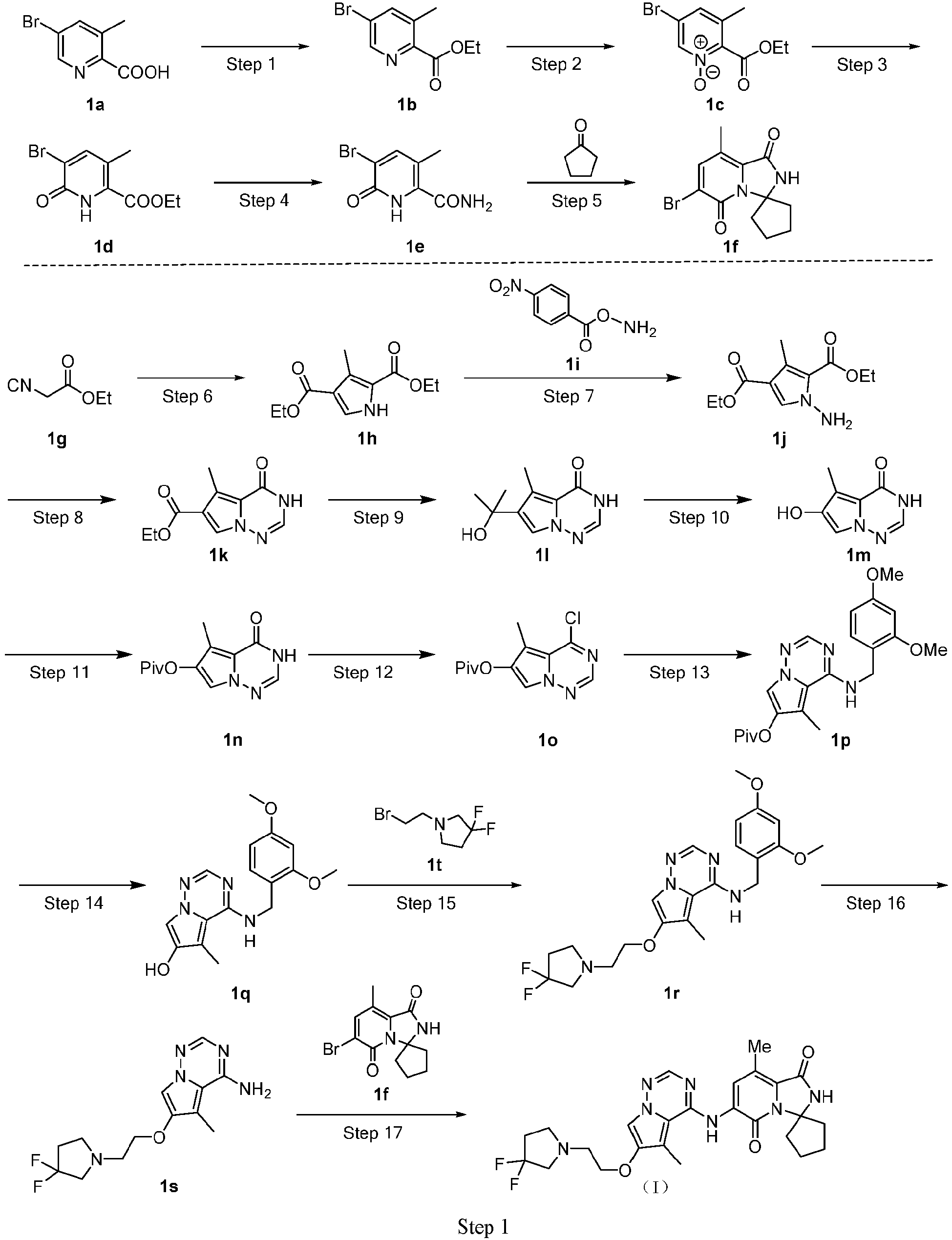

Compound **1a** (100 g, 462 mmol) was dissolved in ethanol (500 mL), and concentrated sulfuric acid (49.94 g, 509 mmol, purity: 98%) was added dropwise at 0 °C. The reaction liquid was stirred at 95 °C for 16 h. After the reaction was completed, the reaction liquid was concentrated under reduced pressure to remove most of the ethanol. Water (300 mL) was added to the concentrate, and the mixed solution was extracted with ethyl acetate (250 mL × 3). The combined organic phase was washed with saturated sodium bicarbonate (300 mL), dried over anhydrous sodium sulfate, and filtered, and the filtrate was concentrated under reduced pressure to give compound **1b**. ¹H NMR (400MHz, CDCl₃) δ 8.60 (s, 1H), 7.78 (s, 1H), 4.48-4.42 (m, 2H), 2.58 (s, 3H), 1.43 (t, *J*=7.2 Hz, 3H).

### Step 2

Compound **1b** (10.0 g, 41.0 mmol) was dissolved in dichloromethane (200 mL), and trifluoroacetic anhydride (17.2 g, 81.9 mmol) and urea hydrogen peroxide (8.09 g, 86.0 mmol) were added under stirring at 0 °C. The reaction liquid was warmed to 25 °C and stirred for 16 h. Water (200 mL) was added to the reaction liquid, and the mixed solution was extracted with dichloromethane (100 mL × 3). The organic phases were combined, washed successively with saturated sodium bicarbonate (200 mL × 2) and saturated brine (500 mL), dried over anhydrous sodium sulfate, filtered, and concentrated under reduced pressure to give compound **1c**. ¹H NMR (400MHz, CDCl₃) δ 8.23 (s, 1H), 7.29 (s, 1H), 4.52-4.45 (m, 2H), 2.29 (s, 3H), 1.41 (t, *J*=7.2 Hz, 3H).

### Step 3

Compound **1c** (22.0 g, 84.6 mmol) was dissolved in *N,N*-dimethylformamide (130 mL), and trifluoroacetic anhydride (35.5 g, 169 mmol) was added under stirring at 0 °C. The reaction liquid was stirred at 50 °C for 1 h. Water (200 mL) was added to the reaction liquid, and the mixed solution was extracted with ethyl acetate (100 mL × 4). The organic phases were combined, washed successively with saturated sodium bicarbonate (200 mL × 3) and saturated brine (150 mL × 3), dried over anhydrous sodium sulfate, filtered, and concentrated under reduced pressure. The crude product was added to a mixed liquid of petroleum ether/ethyl acetate (8/1, 90 mL), and the mixture was stirred at room temperature for 2 h and then filtered to give compound **1d.** ¹H NMR (400MHz, CDCl₃) δ 9.89 (br s, 1H), 7.75 (s, 1H), 4.46-4.41 (m, 2H), 2.44 (s, 3H), 1.43 (t, *J*=7.2 Hz, 3H).

### Step 4

Compound **1d** (2.00 g, 7.69 mmol) was dissolved in ethanol (20 mL), and aqueous ammonia (16.2 g, 115 mmol, purity: 25%) was added. The reaction liquid was stirred at 40 °C for 16 h. The reaction liquid was concentrated under reduced pressure, and the crude product was added to a mixed liquid of methanol/dichloromethane (1/5, 48 mL). The mixture was stirred overnight at room temperature, then filtered and washed with dichloromethane (5 mL × 2). The filter cake was concentrated under reduced pressure to give compound **1e**. MS-ESI, [M+H]⁺, calculated: 231 and 233, found: 231 and 233.

### Step 5

Compound **1e** (500 mg, 1.97 mmol) and cyclopentanone (664 mg, 7.89 mmol) were dissolved in anhydrous dioxane (6 mL), and concentrated sulfuric acid (98.7 mg, 0.986 mmol, purity: 98%) was added dropwise to the reaction liquid. The reaction liquid was stirred at 95 °C for 3 h. The reaction liquid was concentrated under reduced pressure to remove a part of dioxane (about 3 mL), and then filtered. *n*-Hexane (10 mL) was added to the collected filter cake, and the mixture was stirred at room temperature for 2 h and filtered. The filter cake was dried in vacuum for 2 h to give compound **1f**. MS-ESI, [M+H]⁺, calculated: 297 and 299, found: 297 and 299. ¹H NMR (400 MHz, DMSO-*d*₆) δ 10.16 (s, 1H), 8.02 (s, 1H), 2.71-2.78 (m, 2H), 2.37 (s, 3H), 1.91-1.93 (m, 2H), 1.79-1.84 (m, 2H), 1.63-1.67 (m, 2H).

### Step 6

Compound **1g** (50 g, 0.442 mol), 1,8-diazabicyclo[5.4.0]undec-7-ene (67.3 g, 0.442 mol) were dissolved in tetrahydrofuran (500 mL). The reaction liquid was heated to 55 °C, and acetaldehyde (9.74 g, 0.221 mol) was added to the reaction liquid at this temperature. The reaction liquid was stirred at 55 °C for 18 h. Then the reaction liquid was cooled to 22 °C, and quenched with acetic acid (25 mL). The reaction liquid was concentrated under reduced pressure, and the residue was dissolved in ethyl acetate (1000 mL) and diluted hydrochloric acid (1000 mL, 1 M). The organic phase was retained after liquid separation, and the aqueous phase was extracted with ethyl acetate (300 mL × 3). The organic phases were combined, washed successively with saturated sodium bicarbonate solution (100 mL) and brine (200 mL), dried over anhydrous sodium sulfate, filtered, and concentrated, and the crude product was separated by column chromatography (4/1, petroleum ether/ethyl acetate, Rf = 0.56) to give compound **1h**. MS-ESI, [M+H]⁺, calculated: 226, found: 226. ¹H NMR (400 MHz, CDCl₃) δ 9.29 (br s, 1H), 7.48 (d, *J=3.2* Hz, 1H), 4.35 (q, *J*=7.2 Hz, 2H), 4.29 (q, *J*=7.2 Hz, 2H), 2.61 (s, 3H), 1.38 (t, *J*=7.2 Hz, 3H), 1.35 (m, *J*=7.2 Hz, 3H).

### Step 7

Compound **1h** (11.0 g, 48.8 mmol) was dissolved in *N*-methylpyrrolidone (60 mL), and potassium *tert*-butoxide (6.03 g, 53.7 mmol) was added to the reaction liquid. After the reaction liquid was stirred at 25 °C for 0.5 h, a solution of compound **1i** (9.78 g, 53.7 mmol) in N-methylpyrrolidone (30 mL) was added. The reaction liquid was further stirred for 20 h. The reaction liquid was washed with water (200 mL) and extracted with ethyl acetate (200 mL × 3). The organic phases were combined, washed with saturated brine (20 mL × 3), dried over anhydrous sodium sulfate, filtered, and concentrated, and the crude product was separated by column chromatography (4/1, petroleum ether/ethyl acetate, Rf = 0.55) to give compound **1j.** MS-ESI, [M+H]⁺, calculated: 241, found: 241. ¹H NMR (400 MHz, CDCl₃) δ 7.49 (s, 1H), 4.35 (q, *J*=7.2 Hz, 2H), 4.27 (q, *J*=7.2 Hz, 2H), 2.57 (s, 3H), 1.40 (t, *J*=7.2 Hz, 3H), 1.34 (t, *J*=7.2 Hz, 3H).

### Step 8

Compound **1j** (10.2 g, 42.5 mmol) was dissolved in formamide (120 mL), and phosphoric acid (832 mg, 8.49 mmol) was added to the reaction liquid. The reaction liquid was stirred at 125 °C for 16 h. Then the reaction liquid was cooled to 22 °C, and a large amount of white solid was precipitated out here. The mixture was filtered, and the collected filter cake was added to a mixed solution of petroleum ether/ethyl acetate (1/1, 100 mL). The resulting mixture was stirred at 30 °C for 0.5 h and then filtered to give compound **1k**. MS-ESI, [M+H]⁺, calculated: 222, found: 222. ¹H NMR (400 MHz, DMSO-*d*₆) δ 7.90 (s, 1H), 7.84 (s, 1H), 4.23 (q, *J*=7.2 Hz, 2H), 2.61 (s, 3H), 1.28 (t, *J*=7.2 Hz, 3H).

### Step 9

Compound **1k** (4.00 g, 18.0 mmol) was dissolved in anhydrous tetrahydrofuran (50 mL). Methylmagnesium bromide (30.1 mL, 90.3 mmol) was added dropwise to the reaction liquid at 25 °C. After the dropwise addition was completed, the reaction liquid was warmed to 25 °C and stirred for 15 h. The reaction liquid was quenched with saturated ammonium chloride solution (100 mL), and extracted with ethyl acetate (50 mL × 5). The organic phases were combined, washed with saturated brine (10 mL × 1), dried over anhydrous sodium sulfate, filtered, and concentrated under reduced pressure, and the crude product was separated by thin layer chromatography (2/1, petroleum ether/ethyl acetate, Rf = 0.39) to give compound **1l.**

MS-ESI, [M+H]⁺, calculated: 208, found: 208. ¹H NMR (400 MHz, CDCl₃) δ 7.30 (br s, 1H), 7.24 (br s, 1H), 2.59 (s, 3H), 1.54 (s, 6H).

### Step 10

Compound **1l** (1.00 g, 4.83 mmol) and hydrogen peroxide (4.64 mL, 48.26 mmol, content: 30%) were dissolved in anhydrous tetrahydrofuran (30 mL). A cold solution of methanesulfonic acid (3.44 mL, 48.26 mmol) in water (10 mL) was added dropwise to the reaction liquid at 0 °C. The reaction liquid was stirred at 0 °C for 1 h. The reaction liquid was quenched with 10% aqueous sodium sulfite solution (15 mL) until potassium iodide starch test paper showed negative. The solution was extracted with ethyl acetate (50 mL × 3). The organic phases were combined, washed with saturated brine (10 mL × 1), dried over anhydrous sodium sulfate, filtered, and concentrated under reduced pressure, and the crude product was separated by column chromatography (2/1, petroleum ether/ethyl acetate, Rf = 0.38) to give compound **1m.** MS-ESI, [M+H]⁺, calculated: 166, found: 166.

### Step 11

Compound **1m** (400 mg, 2.42 mmol) was dissolved in anhydrous tetrahydrofuran (10 mL), and triethylamine (0.674 mL, 4.84 mmol) and pivaloyl chloride (350 mg, 4.84 mmol) were added to the reaction liquid. The reaction liquid was stirred at 0 °C for 1 h, washed with water (10 mL), and extracted with ethyl acetate (10 mL × 5). The organic phases were combined, washed with saturated brine (10 mL × 1), dried over anhydrous sodium sulfate, filtered, and concentrated under reduced pressure, and the crude product was separated by thin layer chromatography (2/1, petroleum ether/ethyl acetate, Rf = 0.63) to give compound **1n.** MS-ESI, [M+H]⁺, calculated: 250, found: 250. ¹H NMR (400 MHz, CD₃OD) δ 7.61 (s, 1H), 7.47 (s, 1H), 2.34 (s, 3H), 1.38 (s, 9H).

### Step 12

Compound **1n** (450 mg, 1.81 mmol) was dissolved in phosphorus oxychloride (8.85 mL). The reaction liquid was stirred at 100 °C for 1 h. The reaction liquid was then cooled to room temperature and poured into saturated ammonium bicarbonate solution (300 mL). The mixture was extracted with dichloromethane (50 mL × 3), and the organic phases were combined, washed with saturated brine (20 mL × 1), dried over anhydrous sodium sulfate, filtered, and concentrated to give compound **1o**. MS-ESI, [M+H]⁺, calculated: 268, found: 268.

### Step 13

Compound **1o** (2.00 g, 7.47 mmol), 2,4-dimethoxybenzylamine (1.87 g, 11.21 mmol) and triethylamine (2.27 g, 22.4 mmol) were dissolved in anhydrous tetrahydrofuran (30 mL). The reaction liquid was stirred at 70 °C for 1 h. The reaction liquid was then concentrated under reduced pressure to give crude compound **1p.** MS-ESI, [M+H]⁺, calculated: 399, found: 399.

### Step 14

Compound **1p** (3.50 g, 8.78 mmol) was dissolved in methanol (3 mL) and tetrahydrofuran (20 mL), and a solution of sodium hydroxide (703 mg, 17.6 mmol) in water (20 mL) was added to the reaction liquid. The reaction liquid was stirred at 25 °C for 0.5 h. The reaction liquid was concentrated to remove the organic solvent, and the aqueous phase was adjusted to pH = 7 with diluted aqueous hydrochloric acid solution (1 M) and the mixture was extracted with dichloromethane (50 mL × 3). The organic phases were combined, washed with saturated brine (10 mL × 1), dried over anhydrous sodium sulfate, filtered, and concentrated under reduced pressure, and the crude product was separated by column chromatography (2/1, petroleum ether/ethyl acetate, Rf = 0.32) to give compound **1q.** MS-ESI, [M+H]⁺, calculated: 315, found: 315. ¹H NMR (400MHz, CD₃OD) δ 7.67 (s, 1H), 7.21 (d, *J*=8.4 Hz, 1H), 7.07 (s, 1H), 6.56 (d, *J*=2.4 Hz, 1H), 6.47 (dd, *J*=2.4, 8.4 Hz, 1H), 4.66 (s, 2H), 3.90 (s, 3H), 3.79 (s, 3H), 2.36 (s, 3H).

### Step 15

Compound **1t** (250 mg, 795 *µ*mol) was dissolved in *N,N*-dimethylformamide (4 mL), and then compound **1q** (187 mg, 875 *µ*mol) and sodium hydroxide (63.6 mg, 1.59 mmol) were added. The reaction liquid was stirred at 50 °C for 0.5 h. After the reaction was completed, water (50 mL) was added to the reaction liquid for dilution, and the mixed solution was extracted with ethyl acetate (30 mL × 3). The combined organic phase was dried over anhydrous sodium sulfate and filtered, and the filtrate was concentrated under reduced pressure. The residue was separated by column chromatography (1:1, petroleum ether/ethyl acetate, Rf = 0.1) to give compound **1r**. MS-ESI, [M+H]⁺, calculated: 448, found: 448.

### Step 16

Compound **1r** (300 mg, 670 *µ*mol) was dissolved in trifluoroacetic acid (3.0 mL), and the reaction liquid was stirred at 100 °C for 1 h. After the reaction was completed, the reaction liquid was concentrated, and the residue was purified by high performance liquid chromatography (hydrochloric acid system) to give the hydrochloride of compound **1s.** MS-ESI, [M+H]⁺, calculated: 298, found: 298.

### Step 17

The hydrochloride of compound **1s** (90 mg) and compound **1f** (72 mg, 241 *µ*mol) were dissolved in anhydrous dioxane (2 mL), and then cesium carbonate (250 mg, 766 *µ*mol) and methanesulfonate(2-dicyclohexylphosphino-3,6-dimethoxy-2,4,6-tri-isopropyl-1,1-biphenyl)(2-amino-1,1-biphenyl-2-yl)palladium(II) (20 mg, 21.9 *µ*mol) were added. The reaction liquid was stirred at 105 °C for 12 h under nitrogen atmosphere. The reaction liquid was concentrated under reduced pressure, and the residue was separated and purified by column chromatography (10:1, dichloromethane/methanol, Rf = 0.3) to give a crude compound. A mixed solution of methanol and ethanol (4/1, 10 mL) was added to the crude product, and the mixture was stirred at 20 °C for 16 h and filtered. The filter cake was washed with methanol (2 mL × 2) and water (2 mL × 2) and then dried to give a compound of formula (I). MS-ESI, [M+H]⁺, calculated: 514, found: 514. ¹H NMR (400MHz, DMSO-*d*₆) δ 10.00 (s, 1H), 8.84 (s, 1H), 8.64 (s, 1H), 8.08 (s, 1H), 7.70 (s, 1H), 4.08 (t, *J*=5.6 Hz, 2H), 3.00 (t, *J*=13.5 Hz, 2H), 2.91-2.78 (m, 6H), 2.47 (s, 3H), 2.46 (s, 3H), 2.31-2.18 (m, 2H), 2.04-1.92 (m, 2H), 1.91-1.78 (m, 2H), 1.76-1.62 (m, 2H).

### Example 2: Preparation of Compound of Formula (II) and Single Crystal Growth Thereof Synthetic route:

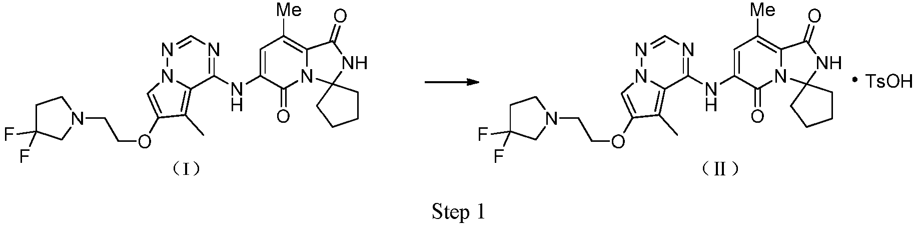

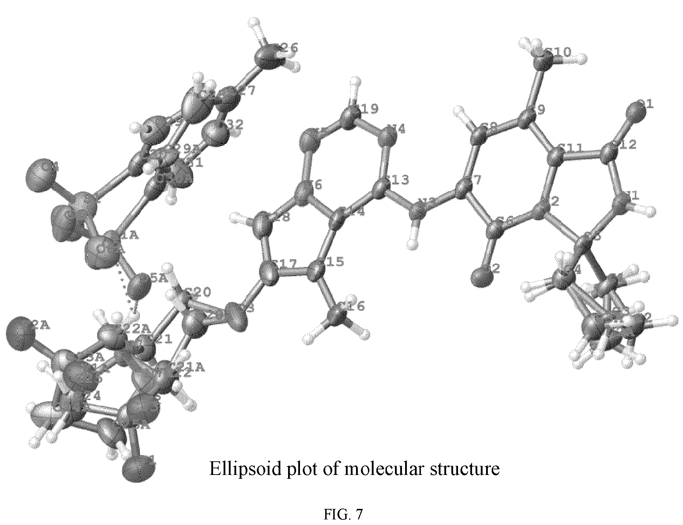

The compound of formula (I) (2 g, 3.89 µmol) and hexafluoroisopropanol (40 mL) were stirred and mixed well, and p-toluenesulfonic acid monohydrate (814.89 mg, 4.28 mmol) was added to the solution. The reaction liquid was stirred at 40 °C for 3 h and added dropwise to isopropanol (160 mL). The mixed solution was filtered, and the filter cake was dried in vacuum to give a compound of formula (II). ¹H NMR (400 MHz, DMSO-*d*₆) δ 10.02 (s, 1H), 8.86 (br s, 1H), 8.64 (s, 1H), 8.11 (s, 1H), 7.78 (s, 1H), 7.48 (d, *J=* 8.0 Hz, 2H), 7.10 (d, *J =* 8.0 Hz, 2H), 4.31 (br d, *J =* 4.4 Hz, 2H), 4.08 - 3.62 (m, 6H), 2.89 - 2.78 (m, 2H), 2.72 - 2.57 (m, 2H), 2.51 (br s, 3H), 2.46 (s, 3H), 2.28 (s, 3H), 1.95-1.97 (m, 2H), 1.89 - 1.79 (m, 2H), 1.73 - 1.64 (m, 2H). MS-ESI, [M+H]⁺, calculated: 514, found: 514. An appropriate amount of the compound of formula (II) was dissolved in 1 mL of dichloromethane/methanol (1:1) at room temperature, and the sample solution was placed in a 4 mL semi-sealed sample bottle to allow the solvents to be slowly volatilized at room temperature. The next day, a colorless massive crystal was obtained and tested by single-crystal X-ray diffraction. The analysis results of the single crystal structure are shown in FIG. 7.

### Example 3: Preparation of Crystal Form A of Compound of Formula (II)

Acetonitrile (7.0 L), the compound of formula (I) (700.22 g), and p-toluenesulfonic acid monohydrate (272.25 g) were added successively to a reactor, with the internal temperature maintained at 20-30 °C. The internal temperature of the reactor was raised to 45-55 °C, and the reaction liquid was stirred for 4.5 h and filtered. The filter cake was rinsed with acetonitrile (0.7 L). The resulting filter cake was transferred into the reactor, acetonitrile (7.0 L) was added, and then the reaction liquid was stirred at 45-55 °C for 20 h and filtered. The filter cake was rinsed successively with acetonitrile (0.7 L) and *tert-butyl* methyl ether (1.4 L), and dried under vacuum and reduced pressure at a temperature not higher than 45 °C and a pressure ≤ -0.1 MPa for 17 h to give crystal form A of the compound of formula (II). The XRPD, DSC, and TGA detection results are shown in FIG. 1, FIG. 2, and FIG. 3.

### Example 4: Crystal Form Screening Test of Compound of Formula (II) in Different Solvents

About 50 mg of the compound of formula (II) was weighed out separately and dissolved in different solvents, the mixtures were stirred at 50 °C for 3 h. The reaction liquids were cooled to room temperature, filtered for collection, and dried in vacuum, and the obtained solids were tested by XRPD. The test results are shown in Table 10 below:

**Table 10. Stability test results of compound of formula (II) in different solvents**

| **No.** | **Solvent** | **Appearance** | **Crystal form** |
|---|---|---|---|
| **1** | Methanol | Suspension | Crystal form A |
| **2** | Acetonitrile | Suspension | Crystal form A |
| **3** | *tert*-Butyl methyl ether | Suspension | Crystal form A |
| **4** | Ethanol | Suspension | Crystal form B |
| **5** | *n*-Heptane | Suspension | Crystal form B |

**Conclusion:** The compound of formula (II) forms crystal form A in methanol, acetonitrile, and *tert-butyl* methyl ether, and forms crystal form B in ethanol and n-heptane, wherein the XRPD and DSC detection results of the crystal form B are shown in FIG. 4 and FIG. 5 in sequence.

### Example 5: Study on Hygroscopicity of Crystal Form A of Compound of Formula (II)

### Materials:

SMS DVS intrinsic dynamic vapor sorption instrument

### Procedures:

The crystal form A of the compound of formula (II) (10-15 mg) was placed in a DVS sample tray for testing.

### Results:

The DVS profile of the crystal form A of the compound of formula (II) is shown in FIG. 6, ΔW = 1.179%.

### Conclusion:

The crystal form A of the compound of formula (II) had a hygroscopic weight gain of 1.179% at 25 °C and 80% RH, had relatively weak hygroscopicity, and was not changed before and after the hygroscopic test, so that the crystal form A is a stable crystal form.

### Experimental Example 6: Study on Solid Stability Test of Crystal Form A of Compound of Present Disclosure

The crystal form A of the compound of the present disclosure was subjected to an accelerated lofting stability test and long-term lofting stability study concerning rules and requirements in "Guidelines for the Stability Test of APIs and Preparations" (General Principles 9001 of the Four Parts of the Chinese Pharmacopoeia, 2020 Edition), "Technical Guidelines for Stability Research of Chemical Drugs (APIs and Preparations) (Revised)" issued by the Center for Drug Evaluation of the National Medical Products Administration, and ICH Q1B.

### 1. Long-term accelerated lofting stability test:

Each sample was separately put into a double-layer LDPE bag with each layer of LDPE bag sealed by a binding buckle. The bag was then put into an aluminum foil bag for heat sealing, and then put into a stability box under corresponding conditions for observation. The results of the solid stability test of the crystal form A of the compound are shown in Table 11.

**Table 11. Results of solid stability test of crystal form A of compound of present disclosure**

| Test conditions | Time point | Crystal form |
|---|---|---|
| 25°C/60%RH | Day 0 | Crystal form A |
| | 12 months | Crystal form A |
| 40°C/75%RH | Day 0 | Crystal form A |
| | 6 months | Crystal form A |

Conclusion: the crystal form A of the compound of the present disclosure has good stability under the conditions of a long-term accelerated lofting stability test, and the crystal form A is a stable crystal form.

### Bioactivity Assay

### Assay Example 1: In Vitro Evaluation of Inhibitory Activity of Compound of Present Disclosure Against MNK2 Protein Kinase

Purpose of experiment: to test the inhibitory activity of the compound against MNK2 protein kinase Experimental materials: assay buffer: 8 mM 3-(*N*-morpholino)propanesulfonic acid, 0.2 mM disodium ethylenediaminetetraacetate, 0.01% polyoxyethylene lauryl ether, 5% glycerol, 0.1% β-mercaptoethanol and 1 mg of bovine serum albumin

Experimental operation: Mnk2 protein kinase inhibitory activity assays were performed using the KinaseProfilerTM service from Eurofins Pharma Discovery Services UK Limited. Serially diluted DMSO solutions containing the compound to be tested (3-fold serial dilution, starting from 10 µM), MNK2 (h) protein kinase and 0.33 mg/mL myelin basic protein were added to a freshly prepared buffer (pH = 7.0), and then stirred homogeneously. The reaction was initiated by adding a mixture of ³³P-ATP (intensity of radioactivity: 10µCi/µL) and 10 mM magnesium acetate. After the resulting mixture was reacted at room temperature for 40 min, the reaction was terminated by adding phosphoric acid to dilute to a concentration of 0.5%. 10 µL of the reaction liquid was filtered using a P30 filtermat, and then the filtermat was washed four times with 0.425% phosphoric acid for 4 min each, followed by washing once with methanol. After drying, the intensity of radioactivity was determined using the Filter-Binding method.

The protein kinase inhibitory activity of the compound was expressed as a percentage of the residual protein kinase activity relative to the blank substrate (DMSO alone). The Prism4 software package (GraphPad) was used to calculate the IC₅₀ value and curve. The specific information is shown in Table 12 below.

**Table 12. Inhibitory activity (IC₅₀) of compound of present disclosure against MNK2 protein kinase**

| **Compound No.** | **IC₅₀ (nM) against MNK2** |
|---|---|
| Compound of formula (I) | 17 |

Experimental conclusion: the compound of formula (I) of the present disclosure shows excellent inhibitory activity against MNK2 protein kinase.

### Assay Example 2: In Vitro Evaluation of Inhibitory Activity of Compound of Present Disclosure Against eIF4E Phosphorylation

Purpose of experiment: to test the inhibition (IC₅₀) of the compound against eIF4E phosphorylation of HCT116 cell strain.

Experimental materials: HCT116 cells (ATCC), RPMl1640 medium (Life technology), fetal bovine serum (Hyclone), double antibodies (penicillin, streptomycin) (Millipore), phosphate buffer (Corning), 384-well cell plate (PerkinElmer), and AlphaLISA^{®} SureFire^{®} Ultra^{™} p-eIF4E (Ser209) Assay Kit (PerkinElmer).

Experimental operation: HCT116 cells were digested to make a cell suspension, which was then plated in a 96-well plate. The cell plate was then placed in an incubator for overnight incubation. The compound was diluted to a corresponding concentration and added to the cell plate, and the resulting mixture was incubated for 3 h. The cells were subsequently lysed with a lysis buffer, and the lysate was transferred to a 3 84-well plate. A mixed receptor was freshly prepared according to the kit instructions and added to the 384-well plate, and the mixture was incubated at room temperature for 1 h. Then a mixed donor was freshly prepared according to the kit instructions and added to the 384-well plate, and the resulting mixture was incubated at room temperature for 1 h. The signal was read on EnVision using the standard AlphaLISA program, a curve was fitted using Graphpad prism, and the IC₅₀ value was calculated. The specific information is shown in Table 13 below.

**Table 13. Inhibitory activity (IC₅₀) of compound of present disclosure against eIF4E phosphorylation**

| **Compound No.** | **IC₅₀ (nM) against HCT116 cell strain p-eIF4E** |
|---|---|
| Compound of formula (I) | 8.6 |

Experimental conclusion: the compound of formula (I) of the present disclosure shows excellent inhibitory activity against eIF4E phosphorylation.

### Assay Example 3: In Vivo Efficacy Experiment of Compound of Present Disclosure for CT-26 Mouse Graft Tumor

Purpose of experiment: to determine *in vivo* efficacy of the compound for CT-26 mouse graft tumor
Experimental materials: CT-26 cells, RMPI-1640 medium containing 10% fetal bovine serum, and mice (female, Shanghai Sippe-Bk Lab Animal Co., Ltd.)
Experimental operation: CT-26 cells were cultured in RMPI-1640 medium containing 10% fetal bovine serum in an incubator at 37 °C/5% CO₂. Tumor cells were subcultured, and after an appropriate concentration was reached and the tumor cells were in the logarithmic growth phase, the tumor cells were collected, counted and then resuspended in DPBS (phosphate buffer), and the concentration of the cell suspension was adjusted to 3×10⁶/mL for inoculation.

Establishment of mouse colon cancer graft tumor: cells were collected and adjusted to a concentration of 3×10⁶ cells/mL (resuspended in DPBS to obtain cell suspension), and 0.1 mL of tumor cells was injected subcutaneously at the right dorsal side of the mice under sterile conditions, and 3×10⁵ cells were inoculated for each mouse. After the tumor grew to a certain size, the length (a) and the width (b) of the tumor were measured by using a digital vernier caliper, and the tumor volume (TV) was calculated, wherein the calculation formula is as follows: TV = a×b²/2.

CT-26 tumor cell inoculation: on the day of inoculation, animals were grouped (8 animals for each) according to the body weight and subjected to drug administration separately, and the day of inoculation was considered as D0. When the tumor size reached about 60 mm³, the animals in the antibody groups were grouped according to the tumor size and the body weight. The body weight and tumor size of the animals were measured three times a week during the experiment, while clinical symptoms of the animals were observed and recorded daily, and for each administration, reference was made to the animal body weight measured most recently. The inhibitory effect of the compound on colon cancer graft tumor in mice was determined after 21-day administration at a dose of 30 mg/Kg QD (once daily), 90 mg/Kg QD (once daily) and 200 mg/Kg QD (once daily). The specific information is shown in Table 14 below.

The evaluation index of the anti-tumor activity is relative tumor proliferation rate T/C (%); if T/C (%) > 40%, it suggests the drug is ineffective, and if T/C (%) ≤ 40% and P < 0.05 after statistical treatment, the drug is considered to be effective. The calculation formula of T/C (%) is as follows: T/C (%) = (T_{RTV}/C_{RTV}) × 100%. T_{RTV} is the relative tumor volume of the treatment group, and C_{RTV} is the relative tumor volume of the negative control group; TGI (%)^{a} = (1 - average tumor volume at the end of administration of a treatment group/average tumor volume at the end of treatment of the solvent control group) × 100%.

**Table 14. In vivo anti-tumor efficacy of compound of present disclosure in CT-26 graft tumor model**

| **Compound** | **Administration dose** | **TGI%** | **T/C%** |
|---|---|---|---|
| Compound of formula (I) | 30mg/Kg, QD | 63.57 | 36.43 |
| Compound of formula (I) | 90mg/Kg, QD | 68.89 | 31.11 |
| Compound of formula (I) | 200mg/Kg, QD | 68.51 | 33.31 |

| | | | |
|---|---|---|---|
| Experimental conclusion: the compound of formula (I) of the present disclosure has a significant effect in inhibiting colon cancer graft tumor in mice. | | | |

## Claims

1. A compound of formula (II),

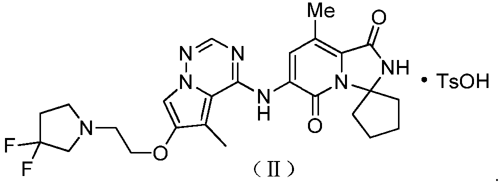

2. Crystal form A of a compound of formula (II), wherein an X-ray powder diffraction pattern of the crystal form A comprises characteristic diffraction peaks at the following 20 angles: 7.78±0.20°, 11.38±0.20°, and 20.58±0.20°, when measured using a Cu Kα radiation;

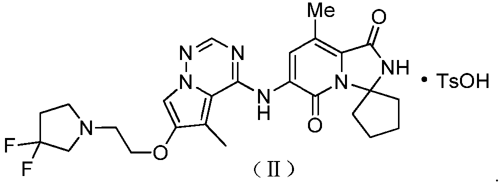

3. The crystal form A according to claim 2, wherein the X-ray powder diffraction pattern of the crystal form A comprises characteristic diffraction peaks at the following 20 angles: 7.78±0.20°, 9.44±0.20°, 11.38±0.20°, 19.84±0.20°, 20.58±0.20°, 21.56±0.20°, 22.86±0.20°, and 24.82±0.20°, when measured using a Cu Kα radiation; or the X-ray powder diffraction pattern of the crystal form A comprises characteristic diffraction peaks at the following 2θ angles: 6.58±0.20°, 7.78±0.20°, 9.44±0.20°, 11.38±0.20°, 14.38±0.20°, 18.66±0.20°, 19.84±0.20°, 20.58±0.20°, 21.56±0.20°, 22.86±0.20°, 23.54±0.20°, and 24.82±0.20°, when measured using a Cu Kα radiation; or the X-ray powder diffraction pattern of the crystal form A comprises characteristic diffraction peaks at the following 2θ angles: 4.78°, 6.58°, 7.78°, 9.44°, 11.38°, 13.48°, 14.38°, 14.80°, 16.42°, 17.00°, 17.32°, 18.34°, 18.66°, 19.08°, 19.60°, 19.84°, 20.28°, 20.58°, 21.56°, 21.84°, 22.52°, 22.86°, 23.26°, 23.54°, 24.46°, 24.82°, 25.50°, 26.04°, 26.58°, 27.42°, 27.82°, 28.07°, 28.42°, 29.08°, 29.66°, 30.08°, 31.20°, 31.42°, 38.22°, and 39.04°, when measured using a Cu Kα radiation;
or the XRPD pattern of the crystal form A is as shown in FIG. 1.

4. The crystal form A according to any one of claims 2 to 3, wherein a differential scanning calorimetry curve of the crystal form A has an endothermic peak at 287.17±3 °C, when measured using scanning speed of 10°C per minute.

5. The crystal form A according to any one of claims 2-4, wherein a DSC profile of the crystal form A is shown in FIG. 2, when measured using scanning speed of 10°C per minute.

6. The crystal form A according to any one of claims 2 to 5, wherein a thermogravimetric analysis curve of the crystal form A shows a weight loss of 0.075% at 200.0±3 °C, when measured using scanning speed of 10°C per minute.

7. The crystal form A according to any one of claims 2-6, wherein a TGA profile of the crystal form A is shown in FIG. 3, when measured using scanning speed of 10°C per minute.

8. A preparation method for crystal form A of the compound of formula (II) according to any one of claims 2-7, comprising the following steps:
(a) adding the compound of formula (II) to a solvent to form a suspension;
(b) stirring the suspension at 40-55 °C for 2-25 h; and
(c) filtering the suspension and then drying the filter cake in vacuum at 30-45 °C for 10-24 h;
wherein the solvent is selected from methanol, acetonitrile, and *tert-butyl* methyl ether.

9. Crystal form B of a compound of formula (II), wherein an X-ray powder diffraction pattern of the crystal form B comprises characteristic diffraction peaks at the following 2θ angles: 7.55±0.20°, 15.13±0.20°, and 19.82±0.20°, when measured using a Cu Kα radiation;

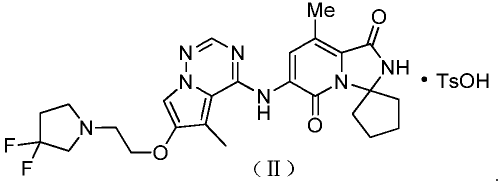

10. The crystal form B according to claim 9, wherein the X-ray powder diffraction pattern of the crystal form B comprises characteristic diffraction peaks at the following 20 angles: 6.89±0.20°, 7.55±0.20°, 9.50±0.20°, 11.35±0.20°, 12.72±0.20°, 15.13±0.20°, 19.82±0.20°, and 26.63±0.20°, when measured using a Cu Kα radiation; or the X-ray powder diffraction pattern of the crystal form B comprises characteristic diffraction peaks at the following 2θ angles: 6.89±0.20°, 7.55±0.20°, 9.50±0.20°, 11.35±0.20°, 12.24±0.20°, 12.72±0.20°, 15.13±0.20°, 18.94±0.20°, 19.82±0.20°, 23.25±0.20°, 26.63±0.20°, and 27.27±0.20°, when measured using a Cu Kα radiation; or the X-ray powder diffraction pattern of the crystal form B comprises characteristic diffraction peaks at the following 20 angles: 4.98°, 6.89°, 7.55°, 8.46°, 9.50°, 10.12°, 11.35°, 12.24°, 12.72°, 14.05°, 15.13°, 15.65°, 16.20°, 17.79°, 18.94°, 19.82°, 20.76°, 21.61°, 23.25°, 23.87°, 26.09°, 26.63°, 27.27°, 28.45°, 29.12°, 30.95°, 32.32°, 34.62°, and 38.41°, when measured using a Cu Kα radiation;
or the XRPD pattern of the crystal form B is as shown in FIG. 4.

11. The crystal form B according to any one of claims 9 -10, wherein a differential scanning calorimetry curve of the crystal form B has an endothermic peak at 300.0±3 °C, when measured using scanning speed of 10°C per minute.

12. The crystal form B according to any one of claim 9-11, wherein a DSC profile of the crystal form B is shown in FIG. 5, when measured using scanning speed of 10°C per minute.

13. The compound of formula (II) according to claim 1, the crystal form A according to any one of claims 2 to 7, or the crystal form B according to any one of claims 9 to 12 for use as a medicament.

14. The compound of formula (II) according to claim 1, the crystal form A according to any one of claims 2 to 7, or the crystal form B according to any one of claims 9 to 12 for use as an MNK1/2 inhibitor medicament.

15. The compound of formula (II) according to claim 1, the crystal form A according to any one of claims 2 to 7, or the crystal form B according to any one of claims 9 to 12 for use in treatment of colorectal cancer.

## Patentansprüche

1. Verbindung der Formel (II),

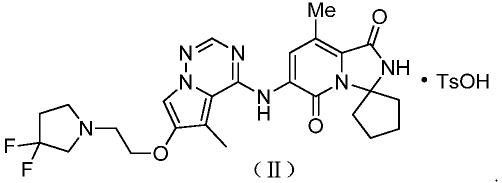

2. Kristallform A einer Verbindung der Formel (II), wobei ein Röntgenpulverbeugungsmuster der Kristallform A charakteristische Beugungspeaks bei den folgenden 2θ-Winkeln umfasst: 7,78 ± 0,20°, 11,38 ± 0,20° und 20,58 ± 0,20°, wenn unter Verwendung einer Cu-Kα-Strahlung gemessen;

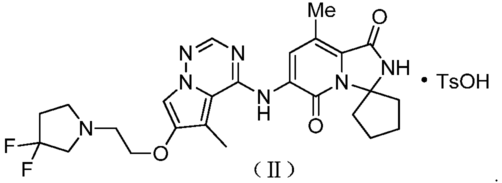

3. Kristallform A nach Anspruch 2, wobei das Röntgenpulverbeugungsmuster der Kristallform A charakteristische Beugungspeaks bei den folgenden 2θ-Winkeln umfasst: 7,78 ± 0,20°, 9,44 ± 0,20°, 11,38 ± 0,20°, 19,84 ± 0,20°, 20,58 ± 0,20°, 21,56 ± 0,20°, 22,86 ± 0,20° und 24,82 ± 0,20°, wenn unter Verwendung einer Cu-Kα-Strahlung gemessen;
oder wobei das Röntgenpulverbeugungsmuster der Kristallform A charakteristische Beugungspeaks bei den folgenden 2θ-Winkeln umfasst: 6,58 ± 0,20°, 7,78 ± 0,20°, 9,44 ± 0,20°, 11,38 ± 0,20°, 14,38 ± 0,20°, 18,66 ± 0,20°, 19,84 ± 0,20°, 20,58 ± 0,20°, 21,56 ± 0,20°, 22,86 ± 0,20°, 23,54 ± 0,20° und 24,82 ± 0,20°, wenn unter Verwendung einer Cu-Kα-Strahlung gemessen;
oder wobei das Röntgenpulverbeugungsmuster der Kristallform A charakteristische Beugungspeaks bei den folgenden 2θ-Winkeln umfasst: 4,78°, 6,58°, 7,78°, 9,44°, 11,38°, 13,48°, 14,38°, 14,80°, 16,42°, 17,00°, 17,32°, 18,34°, 18,66°, 19,08°, 19,60°, 19,84°, 20,28°, 20,58°, 21,56°, 21,84°, 22,52°, 22,86°, 23,26°, 23,54°, 24,46°, 24,82°, 25,50°, 26,04°, 26,58°, 27,42°, 27,82°, 28,07°, 28,42°, 29,08°, 29,66°, 30,08°, 31,20°, 31,42°, 38,22° und 39,04°, wenn unter Verwendung einer Cu-Kα-Strahlunggemessen;
oder wobei das XRPD-Muster der Kristallform A wie in FIG. 1 gezeigt ist.

4. Kristallform A nach einem der Ansprüche 2 bis 3, wobei eine Differential-Scanning-Kalorimetrie-Kurve der Kristallform A einen endothermen Peak bei 287,17 ± 3 °C aufweist, wenn unter Verwendung einer Scangeschwindigkeit von 10 °C pro Minute gemessen.

5. Kristallform A nach einem der Ansprüche 2 bis 4, wobei ein DSC-Profil der Kristallform A in FIG. 2 gezeigt ist, wenn unter Verwendung einer Scangeschwindigkeit von 10 °C pro Minute gemessen.

6. Kristallform A nach einem der Ansprüche 2 bis 5, wobei eine Kurve einer thermogravimetrischen Analyse der Kristallform A einen Gewichtsverlust von 0,075 % bei 200,0 ± 3 °C zeigt, wenn unter Verwendung einer Scangeschwindigkeit von 10 °C pro Minute gemessen.

7. Kristallform A nach einem der Ansprüche 2 bis 6, wobei ein TGA-Profil der Kristallform A in FIG. 3 gezeigt ist, wenn unter Verwendung einer Scangeschwindigkeit von 10 °C pro Minute gemessen.

8. Herstellungsverfahren für die Kristallform A der Verbindung der Formel (II) nach einem der Ansprüche 2 bis 7, das die folgenden Schritte umfasst:
(a) Hinzufügen der Verbindung der Formel (II) zu einem Lösungsmittel, um eine Suspension zu bilden;
(b) Rühren der Suspension bei 40-55 °C für 2-25 h; und
(c) Filtrieren der Suspension und anschließendes Trocknen des Filterkuchens im Vakuum bei 30-45 °C für 10-24 h;
wobei das Lösungsmittel ausgewählt ist aus Methanol, Acetonitril und tert-Butylmethylether.

9. Kristallform B einer Verbindung der Formel (II), wobei ein Röntgenpulverbeugungsmuster der Kristallform B charakteristische Beugungspeaks bei den folgenden 2θ-Winkeln umfasst: 7,55 ± 0,20°, 15,13 ± 0,20° und 19,82 ± 0,20°, wenn unter Verwendung einer Cu-Kα-Strahlung gemessen;

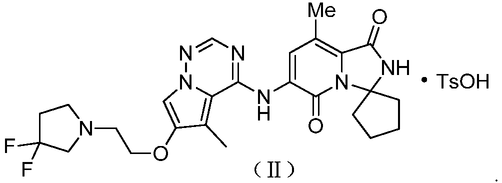

10. Kristallform B nach Anspruch 9, wobei das Röntgenpulverbeugungsmuster der Kristallform B charakteristische Beugungspeaks bei den folgenden 2θ-Winkeln umfasst: 6,89 ± 0,20°, 7,55 ± 0,20°, 9,50 ± 0,20°, 11,35 ± 0,20°, 12,72 ± 0,20°, 15,13 ± 0,20°, 19,82 ± 0,20° und 26,63 ± 0,20°, wenn unter Verwendung einer Cu-Kα-Strahlung gemessen;
oder wobei das Röntgenpulverbeugungsmuster der Kristallform B charakteristische Beugungspeaks bei den folgenden 2θ-Winkeln umfasst: 6,89 ± 0,20°, 7,55 ± 0,20°, 9,50 ± 0,20°, 11,35 ± 0,20°, 12,24 ± 0,20°, 12,72 ± 0,20°, 15,13 ± 0,20°, 18,94 ± 0,20°, 19,82 ± 0,20°, 23,25 ± 0,20°, 26,63 ± 0,20° und 27,27 ± 0,20°, wenn unter Verwendung einer Cu-Kα-Strahlung gemessen;
oder wobei das Röntgenpulverbeugungsmuster der Kristallform B charakteristische Beugungspeaks bei den folgenden 2θ-Winkeln umfasst: 4,98°, 6,89°, 7,55°, 8,46°, 9,50°, 10,12°, 11,35°, 12,24°, 12,72°, 14,05°, 15,13°, 15,65°, 16,20°, 17,79°, 18,94°, 19,82°, 20,76°, 21,61°, 23,25°, 23,87°, 26,09°, 26,63°, 27,27°, 28,45°, 29,12°, 30,95°, 32,32°, 34,62° und 38,41°, wenn unter Verwendung einer Cu-Kα-Strahlung gemessen;
oder wobei das XRPD-Muster der Kristallform B wie in FIG. 4 gezeigt ist.

11. Kristallform B nach einem der Ansprüche 9 bis 10, wobei eine Differential-Scanning-Kalorimetrie-Kurve der Kristallform B einen endothermen Peak bei 300,0 ± 3 °C aufweist, wenn unter Verwendung einer Scangeschwindigkeit von 10 °C pro Minute gemessen.

12. Kristallform B nach einem der Ansprüche 9 bis 11, wobei ein DSC-Profil der Kristallform B in FIG. 5 gezeigt ist, wenn unter Verwendung einer Scangeschwindigkeit von 10 °C pro Minute gemessen.

13. Verbindung der Formel (II) nach Anspruch 1, die Kristallform A nach einem der Ansprüche 2 bis 7 oder die Kristallform B nach einem der Ansprüche 9 bis 12 zur Verwendung als ein Medikament.

14. Verbindung der Formel (II) nach Anspruch 1, die Kristallform A nach einem der Ansprüche 2 bis 7 oder die Kristallform B nach einem der Ansprüche 9 bis 12 zur Verwendung als ein MNK1/2-Inhibitormedikament.

15. Verbindung der Formel (II) nach Anspruch 1, die Kristallform A nach einem der Ansprüche 2 bis 7 oder die Kristallform B nach einem der Ansprüche 9 bis 12 zur Verwendung bei der Behandlung von Dickdarmkrebs.

## Revendications

1. Composé de formule (II),

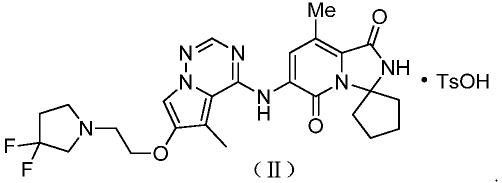

2. Forme cristalline A d'un composé de formule (II), dans laquelle un diagramme de diffraction des rayons X sur poudre de la forme cristalline A comprend des pics de diffraction caractéristiques aux angles 2θ suivants : 7,78 ± 0,20° ; 11,38 ± 0,20° et 20,58 ± 0,20° ; lorsqu'il est mesuré en utilisant un rayonnement Cu Kα ;

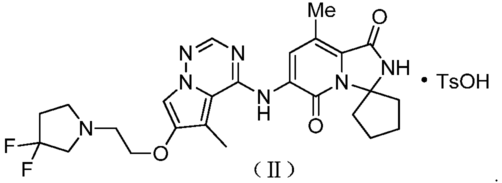

3. Forme cristalline A selon la revendication 2, dans laquelle le diagramme de diffraction des rayons X sur poudre de la forme cristalline A comprend des pics de diffraction caractéristiques aux angles 20 suivants : 7,78 ± 0,20° ; 9,44 ± 0,20° ; 11,38 ± 0,20° ; 19,84 ± 0,20° ; 20,58 ± 0,20° ;21,56 ± 0,20° ; 22,86 ± 0,20° et 24,82 ± 0,20° ; lorsqu'il est mesuré en utilisant un rayonnement Cu Kα ;
ou le diagramme de diffraction des rayons X sur poudre de la forme cristalline A comprend des pics de diffraction caractéristiques aux angles 20 suivants : 6,58 ± 0,20° ; 7,78 ± 0,20° ; 9,44 ± 0,20° ; 11,38 ± 0,20° ; 14,38 ± 0,20° ; 18,66 ± 0,20° ; 19,84 ± 0,20° ; 20,58 ± 0,20° ; 21,56 ± 0,20° ; 22,86 ± 0,20° ; 23,54 ± 0,20° et 24,82 ± 0,20° ; lorsqu'il est mesuré en utilisant un rayonnement Cu Kα ;
ou le diagramme de diffraction des rayons X sur poudre de la forme cristalline A comprend des pics de diffraction caractéristiques aux angles 20 suivants : 4,78° ; 6,58° ; 7,78° ; 9,44° ; 11,38° ; 13,48° ; 14,38° ; 14,80° ; 16,42° ; 17,00° ; 17,32° ; 18,34° ; 18,66° ; 19,08° ; 19,60° ; 19,84° ; 20,28° ; 20,58° ; 21,56° ; 21,84° ; 22,52° ; 22,86° ; 23,26° ; 23,54° ; 24,46° ; 24,82° ; 25,50° ; 26,04° ; 26,58° ; 27,42° ; 27,82° ; 28,07° ; 28,42° ; 29,08° ; 29,66° ; 30,08° ; 31,20° ; 31,42° ; 38,22° et 39,04° ; lorsqu'il est mesuré en utilisant un rayonnement Cu Kα ;
ou le diagramme XRPD de la forme cristalline A est tel que présenté sur la figure 1.

4. Forme cristalline A selon l'une quelconque des revendications 2 à 3, dans laquelle une courbe de calorimétrie différentielle à balayage de la forme cristalline A présente un pic endothermique à 287,17 ± 3 °C, lorsqu'elle est mesurée en utilisant une vitesse de balayage de 10 °C par minute.

5. Forme cristalline A selon l'une quelconque des revendications 2 à 4, dans laquelle un profil DSC de la forme cristalline A est présenté sur la figure 2, lorsqu'elle est mesurée en utilisant une vitesse de balayage de 10 °C par minute.

6. Forme cristalline A selon l'une quelconque des revendications 2 à 5, dans laquelle une courbe d'analyse thermogravimétrique de la forme cristalline A montre une perte de poids de 0,075 % à 200,0 ± 3 °C, lorsqu'elle est mesurée en utilisant une vitesse de balayage de 10 °C par minute.

7. Forme cristalline A selon l'une quelconque des revendications 2 à 6, dans laquelle un profil TGA de la forme cristalline A est présenté sur la figure 3, lorsqu'elle est mesurée en utilisant une vitesse de balayage de 10 °C par minute.

8. Procédé de préparation de la forme cristalline A du composé de formule (II) selon l'une quelconque des revendications 2 à 7, comprenant les étapes suivantes :
(a) l'ajout du composé de formule (II) à un solvant pour former une suspension ;
(b) l'agitation de la suspension à 40 à 55 °C pendant 2 à 25 h ; et
(c) la filtration de la suspension puis le séchage du gâteau de filtration sous vide à 30 à 45 °C pendant 10 à 24 h ;
dans lequel le solvant est choisi parmi méthanol, acétonitrile et méthyl *tert-butyl* éther.

9. Forme cristalline B d'un composé de formule (II), dans laquelle un diagramme de diffraction des rayons X sur poudre de la forme cristalline B comprend des pics de diffraction caractéristiques aux angles 2θ suivants : 7,55 ± 0,20° ; 15,13 ± 0,20° et 19,82 ± 0,20° ; lorsqu'il est mesuré en utilisant un rayonnement Cu Kα ;

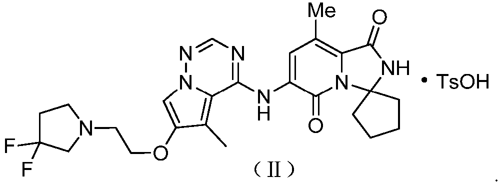

10. Forme cristalline B selon la revendication 9, dans laquelle le diagramme de diffraction des rayons X sur poudre de la forme cristalline B comprend des pics de diffraction caractéristiques aux angles 20 suivants : 6,89 ± 0,20° ; 7,55 ± 0,20° ; 9,50 ± 0,20° ; 11,35 ± 0,20° ; 12,72 ± 0,20° ; 15,13 ± 0,20° ; 19,82 ± 0,20° et 26,63 ± 0,20° ; lorsqu'il est mesuré en utilisant un rayonnement Cu Kα ;
ou le diagramme de diffraction des rayons X sur poudre de la forme cristalline B comprend des pics de diffraction caractéristiques aux angles 20 suivants : 6,89 ± 0,20° ; 7,55 ± 0,20° ; 9,50 ± 0,20° ; 11,35 ± 0,20° ; 12,24 ± 0,20° ; 12,72 ± 0,20° ; 15,13 ± 0,20° ; 18,94 ± 0,20° ; 19,82 ± 0,20° ; 23,25 ± 0,20° ; 26,63 ± 0,20° et 27,27 ± 0,20° ; lorsqu'il est mesuré en utilisant un rayonnement Cu Kα ;
ou le diagramme de diffraction des rayons X sur poudre de la forme cristalline B comprend des pics de diffraction caractéristiques aux angles 20 suivants : 4,98° ; 6,89° ; 7,55° ; 8,46° ; 9,50° ; 10,12° ; 11,35° ; 12,24° ; 12,72° ; 14,05° ; 15,13° ; 15,65° ; 16,20° ; 17,79° ; 18,94° ; 19,82° ; 20,76° ; 21,61° ; 23,25° ; 23,87° ; 26,09° ; 26,63° ; 27,27° ; 28,45° ; 29,12° ; 30,95° ; 32,32° ; 34,62° et 38,41° ; lorsqu'il est mesuré en utilisant un rayonnement Cu Kα ;
ou le diagramme XRPD de la forme cristalline B est tel que présenté sur la figure 4.

11. Forme cristalline B selon l'une quelconque des revendications 9 à 10, dans laquelle une courbe de calorimétrie différentielle à balayage de la forme cristalline B présente un pic endothermique à 300,0 ± 3 °C, lorsqu'elle est mesurée en utilisant une vitesse de balayage de 10 °C par minute.

12. Forme cristalline B selon l'une quelconque des revendications 9 à 11, dans laquelle un profil DSC de la forme cristalline B est présenté sur la figure 5, lorsqu'elle est mesurée en utilisant une vitesse de balayage de 10 °C par minute.

13. Composé de formule (II) selon la revendication 1, forme cristalline A selon l'une quelconque des revendications 2 à 7, ou forme cristalline B selon l'une quelconque des revendications 9 à 12 pour une utilisation en tant que médicament.

14. Composé de formule (II) selon la revendication 1, forme cristalline A selon l'une quelconque des revendications 2 à 7, ou forme cristalline B selon l'une quelconque des revendications 9 à 12 pour une utilisation en tant que médicament inhibiteur de MNK1/2.

15. Composé de formule (II) selon la revendication 1, forme cristalline A selon l'une quelconque des revendications 2 à 7, ou forme cristalline B selon l'une quelconque des revendications 9 à 12 pour une utilisation dans le traitement du cancer colorectal.
